# EUROPEAN PATENT SPECIFICATION

(11) **EP 2 076 601 B1**
(45) Date of publication and mention of the grant of the patent: **30.03.2011**
(21) Application number: 07820804.8
(22) Date of filing: 02.10.2007
(51) Int. Cl.: C12N 15/52

(54) **HIGH YIELD PRODUCTION OF SIALIC ACID (NEU5AC) BY FERMENTATION**
FERMENTATIVE PRODUKTION VON SIALINSÄURE (NEU5AC) MIT HOHER AUSBEUTE
PRODUCTION A HAUT RENDEMENT D'ACIDE SIALIQUE (NEU5AC) PAR FERMENTATION

(30) Priority: 03.10.2006 US 848645 P
(43) Date of publication of application: 08.07.2009
(73) Proprietor: CENTRE NATIONAL DE LA RECHERCHE SCIENTIFIQUE (CNRS), 75016 Paris (FR)
(72) Inventor: SAMAIN, Eric, 38610 Gières (FR)
(74) Representative: Warcoin, Jacques
(86) International application number: PCT/EP2007/060422
(87) International publication number: WO 2008/040717

(56) References cited:
- EP-A- 1 484 406
- WO-A-2006/034225
- RINGENBERG MICHAEL A ET AL: "The first committed step in the biosynthesis of sialic acid by Escherichia coli K1 does not involve a phosphorylated N-acetylmannosamine intermediate." MOLECULAR MICROBIOLOGY, vol. 50, no. 3, November 2003 (2003-11), pages 961-975, XP002472784 ISSN: 0950-382X
- TANNER M E: "The enzymes of sialic acid biosynthesis" BIOORGANIC CHEMISTRY, ACADEMIC PRESS INC., NEW YORK, NY, US, vol. 33, no. 3, June 2005 (2005-06), pages 216-228, XP004879644 ISSN: 0045-2068

### SEQUENCE LISTING

<110> CENTRE NATIONAL DE LA RECHERCHE SCIENTIFIQUE (CNRS) SAMAIN, Eric
<120> High yield production of sialic acid (Neu5Ac) by fermentation
<130> D24440
<150> US 60/848,645
   <151> 2006-10-03
<160> 2
<170> PatentIn version 3.3
<210> 1
   <211> 58
   <212> DNA
   <213> Artificial sequence
<220>
   <223> Upstream primer
<400> 1
   ggcagaacag gcgggcgcgg ttgccattcg cattgaaggt gtaggctgga gctgcttc 58
<210> 2
   <211> 58
   <212> DNA
   <213> Artificial sequence
<220>
   <223> Downstream primer
<400> 2
   ctcgtcaccc tgcccggcgc gcgtgaaaat agttttcgca tatgaatatc ctccttag 58

## Description

### FIELD OF THE INVENTION

The present invention relates to a method for producing sialic acid (Neu5Ac), comprising the step of culturing a microorganism in a culture medium, wherein said microorganism comprises heterologous genes encoding a sialic acid synthasc (NcuB), a UDP-GlcNAc epimerase (NeuC), said micro-organism being devoid of a gene encoding CMP-Neu5Ac synthase (NeuA) or wherein a gene encoding CMP-Neu5Ac synthase (NcuA) has been inactivated or deleted; and wherein endogenous genes coding for sialic acid aldolase (NanA), for ManNac kinase (NanK) and for sialic acid transporter (NanT) have been deleted or inactivated. It also relates to according microorganisms according to claim 12.

### BACKGROUND OF THE INVENTION

N-acctylncuraminic acid (Ncu5Ac) is the most widespread sugar of the sialic acid family whose members are frequently found as a terminal sugar in cell surface complex carbohydrates and are known to play a major role in many processes of biological recognition such as cellular adhesion and binding of toxins and virus (Varki, 1993). All sialic acids are biosynthetically derived from Neu5Ac by the introduction of various modifications such as methylation, acetylation or sulfation. Hydroxylation of the acetyl group of Ncu5Ac leads to the formation of a distinct branch of sialic acid called *N-*glycolylneuraminic acid (Neu5Gc).

In reason of the central role of Neu5Ac in sialic acids metabolism and of its potential utilization for the synthesis of biologically active sialylated oligosaccharides, there has long been a strong interest in developing economic and efficient methods for Neu5Ac preparation. Ncu5Ac used to be purified from animal sources such as edible bird's ncst (Martin *et al.,* 1977) or egg yolk (Koketsu *et al.,* 1992). However the low sialic acid content of these materials resulted in a low overall production yield and precluded the development of an economically practical industrial process. Neu5Ac has also been produced by enzymatic hydrolysis of colominic acid which is an homopolymer of Neu5Ac secreted by strains of *Escherichia coli* K1 (Uchida *et al.,* 1973).

An alternative is the enzymatic synthesis of Neu5Ac from *N*-acetylmannosamine (ManNAc) and pyruvate using the *N*-acetylneuraminic acid aldolase. This enzyme has been identified in various microorganisms and physiologically acts as an aldolase to enable the catabolism of Neu5Ac. The reaction is reversible and the equilibrium can be shifted toward the synthesis of Ncu5Ac in presence of an excess of pyruvate. ManNAc is an expensive compound which is normally prepared by epimerization of *N-*acetylglucosamine (GlcNAc) under alkaline condition (Blayer *et al.,* 1999; Mahmoudian *et al.,* 1997). This epimerization can also be catalyzed by the GlcNAc-2 epimerase which has been advantageously coupled with the Neu5Ac aldolase to directly produce Neu5Ac in one step from GlcNAc and pyruvate (Kragl *et al.,* 1991). The GlcNAc-2 epimerase has been cloned and identified as the renin-binding protein from porcine kidney (Maru *et al.,* 1996). Its gene has been successfully expressed in *E. coli* allowing a better access to the enzyme and the development of different processes of Neu5Ac production (Lee *et al.,* 2004; Maru *et al.,* 1998).

WO 2006/034225 describes production of complex oligosaccharides including sialylated saccharides by micro-organisms having an active NeuA gene. Further, this document does not describe production of sialic acid by micro-organisms.

EP 1 484 406 relates to the production of sialic acid in bacteria comprising an inactivated NanA gene. This document contains no teaching with respect to the role of the NeuA gene.

In spite of these successive improvements the manufacturing cost of Neu5Ac is still relatively high and we have investigated the possibility of reducing this cost by producing Neu5Ac by bacterial fermentation.

In connection with the present invention, we discovered that it is possible to produce genetically engineered micro-organisms, especially non-pathogenic bacteria, by introducing several heterologous genes and inactivating several endogenous genes to obtain a tailored enzymatic pathway leading to accumulation of endogenous sialic acid. In addition, using a strain devoid of sialic acic transporter or by inactivation of endogenous sialic acic transporter gene, we have demonstrated that our living factory is capable of producing high level sialic acid in the culture media without the need of cell lysis. At last, such genetically engineered micro-organisms are not only viable but they are able to grow in standard conditions.

### BRIEF SUMMARY OF THE INVENTION

The present invention provides a method of producing sialic acid by fermentative growth of microorganisms.

### DESCPRIPTION OF THE DRAWINGS

**Figure 1** Relation between catabolic and anabolic pathway of Neu5Ac. Dotted lines represent the enzymatic reactions that have been abolished to make possible the production of Neu5Ac by bacterial fermentation.
**Figure 2** Production of Neu5Ac by long term high cell density cultures of strain SI2 with a glycerol feeding rate of 3.15 g.h⁻¹L⁻¹ (**A**) and 4.2 g.h⁻¹L⁻¹ (B). (■) extracellular Neu5Ac; (□) intracellular Neu5Ac; (-) bacterial growth.

### DISCLOSURE OF ORIGIN OF GENETIC MATERIAL

**Table 1. Exemplary genes, plasmids and Escherichia coli strains used in present invention**

| | Description | Reference or source |
|---|---|---|
| ***Genes*** | | |
| *neuA* | CMP-Neu5Ac synthetase from *C. jejuni* strain ATCC 43438 | AF400048 |
| *neuB* | Sialic acid synthase from C. *jejuni* strain ATCC 43438 | AF400048 |
| *neuC* | GlcNAc-6-phosphate 2 epimerase from *C*. *jejuni* strain ATCC 43438 | AF400048 |
| | | |

### DETAILED DESCRIPTION OF THE INVENTION

In both animals and bacteria, the biosynthesis of Neu5Ac is initiated by UDP-GlcNAc 2-epimerase, which forms ManNAc from UDP-GlcNAc. In animals ManNAc is then phosphorylated at C-6 by a specific ManNAc kinase; ManNAc-6-P is metabolized further by Neu5Ac-9-phosphate synthase to Neu5Ac 9-phosphate which is then dephophorylated into Neu5Ac.

By contrast in bacteria, ManNAc is used instead of ManNAc-6-P for the condensation with phosphoenolpyruvate leading to the formation of Neu5Ac in only one step. The genes *neuC* and *neuB* encoding UDP-GlcNAc 2-epimerase (Vann *et al.,* 2004) and Neu5Ac synthase (Annunziato *et al.,* 1995; Vann *et al.,* 1997) respectively have been identified in *E. coli* K1 and orthologs of these genes have found in various microorganisms such as *Neisseria* and *Campylobacter* species. Thus, the terms *neuC,* and *neuC* are used herein to refer to *E. coli* genes, their orthologs in *Neisseria* and *Campylobacter* species, as well as other bacterial species, mammals and fungi, such as yeast.

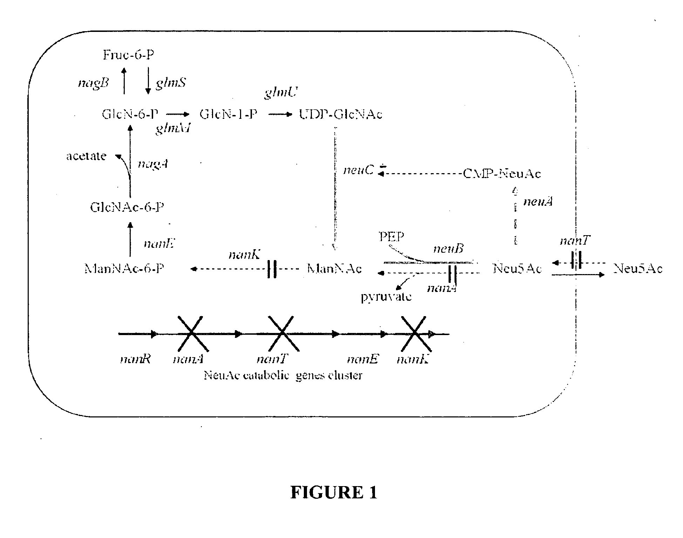

To serve as a substrate for the sialyltransferases Neu5Ac is activated into CMP-Neu5Ac by CMP-Neu5Ac synthase. In animals, the CMP-Neu5Ac biosynthesis flux is regulated by the activity of the UDP-GlcNAc 2-epimerase which has been shown to be feedback-inhibited by CMP-Neu5Ac (Kornfeld *et al.,* 1964). In sialuria, a sialic acid storage disorder, free sialic acid accumulates due to a defect in the regulation of UDP-GlcNAc 2-epimerase by CMP-Neu5Ac. The mechanism of regulation of CMP-Neu5Ac biosynthesis in bacteria has not been determined. However, bacterial UDP-GlcNAc 2-epimerases show high sequence similarities with their animal counterparts and we found that a similar mechanism of feedback inhibition by CMP-Neu5Ac also exists in bacteria. Thus, the expression of *neuB* and *neuC* genes in a bacteria results in an accumulation of Neu5Ac if the bacteria is devoid of CMP-Neu5Ac synthase activity. We genetically engineered non pathogenic strains to produce Neu5Ac from endogenous UDP-GlcNAc by expressing *neuB* and *neuC* genes without expressing a gene for CMP-Ncu5Ac synthase (Figure 1).

In addition, many bacteria including E. coli K12 are able to catabolise Neu5Ac and use it as a carbon energy source. The catabolic pathway for Neu5Ac has been identified in *E. coli :* a specific permease encoded by *nanT* transports Neu5Ac into the cytoplasm, where it is cleaved into ManNAc and pyruvate by the aldolase encoded by *nanA* (Vimr & Troy, 1985). ManNAc is phosphorylated by the NanK kinase into NanNAc-6-P, which is subsequently converted into GlcNAc-6-P by the NanE protein (Plumbridge & Vimr, 1999) GlcNAc-6-P is then deacetylated by NagA into GlcN-6-P to join the glycolysis pathway or to be used as a precursor for UDP-GlcNAc biosynthesis. The *nanT, nanA, nanK* and *nanE* genes are part of the same operon, which is regulated by the DNA binding protein NanR and induced by Neu5Ac (Kalivoda *et al.,* 2003). The production of Neu5Ac by the NeuB and NeuC proteins can thus induce the pathway of Neu5Ac catabolism and create two futile cycles that reduce the capacity of CMP-Neu5Ac biosynthesis of the bacteria. A first futile cycle can result from the combined activity of the sialic acid synthase NeuB with the sialic acid aldolase NanA. A second futile cycle can result from the combined action of the UDP-GlcNAc 2 epimerase NeuC with the four enzymes NanK NanE NagA GlmM and GlmU that catalyse the formation of UDP-GlcNAc from ManNAc. We prevented the degradation of Neu5Ac and ManNAc which are formed by the activity of NeuC and NeuB . We found that this can be advantageously done by disrupting the *nanA* and *nanK* genes in the strains which will be used for Neu5Ac production.

Neu5Ac is a relatively small molecule which is very likely to diffuse into the extracellular medium after being produced in the cytoplasm. Strains expressing a functional Neu5Ac permease are thus expected to continuously re-internalize the Neu5Ac which diffuse in the extracellular medium, creating a futile cycle which could be deleterious to the cells. This can be avoided by disrupting the *nanT* gene which has been shown to encode Neu5Ac permease in *E*. *coli* (Martinez *et al.,* 1995).

With such final strain with all the above modifications, we ended with high scale production of sialic acid reaching up to about 40 g/l under optimized cultured conditions. Such results allow for the first time to produce sialic acid at low commercial cost.

In a first embodiment, the invention relates to a method for producing sialic acid and analogs thereof, comprising the step consisting of culturing a microorganism in a culture medium, wherein said microorganism comprises heterologous genes encoding a sialic acid synthase (NeuB), a UDP-GlcNAc epimerase (NeuC), said micro-organism being devoid of a gene encoding CMP-Neu5Ac synthase (NeuA) or wherein a gene encoding CMP-Neu5Ac synthase (NeuA) has been inactivated or deleted; and wherein endogenous genes coding for sialic acid aldolase (NanA), for sialic acid transporter (nanT), and optionally for ManNac kinase (nanK), have been deleted or inactivated.

According to the method proposed herein, degradation of Ncu5Ac and ManNAc is prevented. This can be advantageously done by disrupting the *nanA* and *nanK* genes. Since deletion or inactivation of *nanT* is also required, the method can be practiced by removing the complete operon for example. Indeed, the *nanT, nanA, nanK* and *nanE* genes are part of the same operon, which is regulated by the DNA binding protein NanR and induced by Neu5Ac (Kalivoda *et al.,* 2003). Thus, the microorganisms of the invention can also be *nanKEAT-.*
Alternatively, the method may comprise removing the operon including *nanT, nanA. nanE* genes (*nanEAT*-), except the *nanK* gene. Thus, the microorganisms of the invention can also be *nanEAT*-.

It will be understood that heterologous genes that can be introduced may originate from different sources, such as *E. coli, Neisseria, Campylobacter* species, as well as mammals or yeasts. Preferably, NeuB and NeuC are isolated from bacterial strains that contain sialylated structure in their cells envelope, such as *C*. *jejuni* strain ATCC Accession No. 43438.

In the above method, micro-organism can be culture in conditions comprising an exponential growth phase which starts with the inoculation of the fermenter and lasts until exhaustion of the carbon substrate (for example glucose at 17.5g.L⁻¹). Preferably, after this first step, micro-organisms are grown in a 40 hours to at least a 75, 100 or 150 hours fed-batch with a high glycerol feeding rate of between 4 g.L⁻¹h⁻¹ to 6 g.L⁻¹h⁻¹. This allows maximization of the level of sialic acid produced in the culture medium. In addition, the method may comprise steps of purification such as removal of cells by centrifugation, followed by crystallization and filtration.

The invention also relates to the microorganism of claim 12 and to a cell culture medium comprising this microorganism and sialic acid produced therefrom which concentration ranges from 10 to 50 g/l in said culture medium.

### DEFINITIONS

The term "sialic acid" refers to any member of a family of nine-carbon carboxylated sugars. The most common member of the sialic acid family is N-acetyl-neuraminic acid (2-keto-5-acetamido-3,5-dideoxy-D-glycero-D-galactononulopyranos-1-onic acid (often abbreviated as Ncu5Ac, Ncu5Ac, or NANA). A second member of the family is N-glycolyl-neuraminic acid (Neu5Gc or NeuGc), in which the N-acetyl group of Neu5Ac is hydroxylated. A third sialic acid family member is 2-keto-3-deoxy-nonulosonic acid (KDN) (Nadano et al. (1986) J. Biol. Chem. 261: 11550-11557; Kanamori et al., J. Biol. Chem. 265: 21811-21819 (1990)). Also included are 9-substituted sialic acids such as a 9-O-C₁-C₆ acyl-Neu5Ac like 9-O-lactyl-Ncu5Ac or 9-O-acetyl-Ncu5Ac 9-deoxy-9-fluoro-Neu5Ac and 9-azido-9-deoxy-Neu5Ac. For review of the sialic acid family, *see, e.g*., Varki, Glycobiology 2: 25-40 (1992); Sialic Acids: Chemistry, Metabolism and Function, R. Schauer, Ed. (Springer-Verlag, New York (1992)). The synthesis and use of sialic acid compounds in a sialylation procedure is disclosed in international application WO 92/16640, published October 1, 1992.

A "culture medium" refers to any liquid, semi-solid or solid media that can be used to support the growth of a microorganism used in the methods of the invention. In some embodiments, the microorganism is a bacterium, *e.g., E. coli.* Media for growing microorganisms arc well known, see, *e*.*g*., Sambrook et al. and Current Protocols in Molecular Biology, F.M. Ausubel et al., eds., Current Protocols, a joint venture between Greene Publishing Associates, Inc. and John Wiley & Sons, Inc., (1998 Supplement) (Ausubel). Media can be rich media, *e*.*g*., Luria broth or terrific broth, or synthetic or semi-synthetic medium, *e.g*., M9 medium. In some preferred embodiments the growth medium comprises lactose and sialic acid.

"Commercial scale" refers to gram scale production of a sialic acid in a single reaction. In preferred embodiments, commercial scale refers to production of greater than about 50, 75, 80, 90 or 100, 125, 150, 175, or 200 grams.

The term "operably linked" refers to functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence.

A "heterologous polynucleotide" or a "heterologous gene", as used herein, is one that originates from a source foreign to the particular host cell, or, if from the same source, is modified from its original form. Thus, a heterologous sialyltransferase gene in a cell includes a gene that is endogenous to the particular host cell but has been modified. Modification of the heterologous sequence may occur, *e.g*., by treating the DNA with a restriction enzyme to generate a DNA fragment that is capable of being operably linked to a promoter. Techniques such as site-directed mutagenesis are also useful for modifying a heterologous sequence.

A "recombinant expression cassette" or simply an "expression cassette" is a nucleic acid construct, generated recombinantly or synthetically, with nucleic acid elements that are capable of affecting expression of a structural gene in hosts compatible with such sequences. Expression cassettes include at least promoters and optionally, transcription termination signals. Typically, the recombinant expression cassette includes a nucleic acid to be transcribed (*e*.*g*., a nucleic acid encoding a desired polypeptide), and a promoter. Additional factors necessary or helpful in effecting expression may also be used. Transcription termination signals, enhancers, and other nucleic acid sequences that influence gene expression, can also be included in an expression cassette. When more than one heterologous protein is expressed in a microorganism, the genes encoding the proteins can be expressed on a single expression cassette or on multiple expression cassettes that are compatible and can be maintained in the same cell. As used herein, expression cassette also encompasses nucleic acid constructs that are inserted into the chromosome of the host microorganism. Those of skill are aware that insertion of a nucleic acid into a chromosome can occur, *e.g*., by homologous recombination. An expression cassette can be constructed for production of more than one protein. The proteins can be regulated by a single promoter sequence, as for example, an operon. Or multiple proteins can be encoded by nucleic acids with individual promoters and ribosome binding sites.

The term "isolated" refers to material that is substantially or essentially free from components which interfere with the activity biological molecule. For cells, saccharides, nucleic acids, and polypeptides of the invention, the term "isolated" refers to material that is substantially or essentially free from components which normally accompany the material as found in its native state. Typically, isolated saccharides, oligosaccharides, proteins or nucleic acids of the invention are at least about 50%, 55%, 60%, 65%, 70%, 75%, 80% or 85% pure, usually at least about 90%, 91%, 92%, 93%, 94%, 95%, 96%, 91%, 98%, or 99% pure as measured by band intensity on a silver stained gel or other method for determining purity. Purity or homogeneity can be indicated by a number of means well known in the art, such as polyacrylamide gel electrophoresis of a protein or nucleic acid sample, followed by visualization upon staining. For certain purposes high resolution will be needed and HPLC or a similar means for purification utilized. For oligosaccharides, *e.g*., sialylated products, purity can be determined using, *e.g*., thin layer chromatography, HPLC, or mass spectroscopy.

The terms "identical" or percent "identity," in the context of two or more nucleic acid or polypeptide sequences, refer to two or more sequences or subsequences that are the same or have a specified percentage of amino acid residus or nucleotides that are the same, when compared and aligned for maximum correspondence, as measured using one of the following sequence comparison algorithms or by visual inspection.

The phrase "substantially identical," in the context of two nucleic acids or polypeptides, refers to two or more sequences or subsequences that have at least 60%, preferably 80% or 85%, most preferably at least 90%, 91%, 92%, 93%, 94%, 95%, 96%, 97%, 98%, or 99% nucleotide or amino acid residu identity, when compared and aligned for maximum correspondence, as measured using one of the following sequence comparison algorithms or by visual inspection. Preferably, the substantial identity exists over a region of the sequences that is at least about 50 residus in length, more preferably over a region of at least about 100 residus, and most preferably the sequences are substantially identical over at least about 150 residus. In a most preferred embodiment, the sequences are substantially identical over the entire length of the coding regions.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection *(see generally,* Current Protocols in Molecular Biology, F.M. Ausubel et al., eds., Current Protocols, a joint venture between Greene Publishing Associates, Inc. and John Wiley & Sons, Inc., (1995 Supplement) (Ausubel)).

Examples of algorithms that are suitable for determining percent sequence identity and sequence similarity are the BLAST and BLAST 2.0 algorithms, which are described in Altschul et al. (1990) J. Mol. Biol. 215: 403-410 and Altschuel et al. (1977) Nucleic Acids Res. 25: 3389-3402, respectively. Software for performing BLAST analyses is publicly available through the National Center for Biotechnology Information (www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al, supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residus; always > 0) and N (penalty score for mismatching residus; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residu alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix *(see* Henikoff & Henikoff, Proc. Natl. Acad. Sci. USA 89:10915 (1989)).

In addition to calculating percent sequence identity, the BLAST algorithm also performs a statistical analysis of the similarity between two sequences *(see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

"Conservatively modified variations" of a particular polynucleotide sequence refers to those polynucleotides that encode identical or essentially identical amino acid sequences, or where the polynucleotide does not encode an amino acid sequence, to essentially identical sequences. Because of the degeneracy of the genetic code, a large number of functionally identical nucleic acids encode any given polypeptide. For instance, the codons CGU, CGC, CGA, CGG, AGA, and AGG all encode the amino acid arginine. Thus, at every position where an arginine is specified by a codon, the codon can be altered to any of the corresponding codons described without altering the encoded polypeptide. Such nucleic acid variations are "silent substitutions" or "silent variations," which are one species of "conservatively modified variations." Every polynucleotide sequence described herein which encodes a polypeptide also describes every possible silent variation, except where otherwise noted. Thus, silent substitutions are an implied feature of every nucleic acid sequence which encodes an amino acid. One of skill will recognize that each codon in a nucleic acid (except AUG, which is ordinarily the only codon for methionine) can be modified to yield a functionally identical molecule by standard techniques. In some embodiments, the nucleotide sequences that encode the enzymes are preferably optimized for expression in a particular host cells.

Similarly, "conservative amino acid substitutions," in one or a few amino acids in an amino acid sequence are substituted with different amino acids with highly similar properties are also readily identified as being highly similar to a particular amino acid sequence, or to a particular nucleic acid sequence which encodes an amino acid. Such conservatively substituted variations of any particular sequence are a feature of the present invention. Individual substitutions, deletions or additions which alter, add or delete a single amino acid or a small percentage of amino acids (typically less than 5%, more typically less than 1%) in an encoded sequence are "conservatively modified variations" where the alterations result in the substitution of an amino acid with a chemically similar amino acid. Conservative substitution tables providing functionally similar amino acids are well known in the art. *See, e.g.,* Creighton (1984) Proteins, W.H. Freeman and Company.

### HOST CELLS

The recombinant cells of the invention are generally made by creating or otherwise obtaining a polynucleotide that encodes the particular enzyme(s) of interest, placing the polynucleotide in an expression cassette under the control of a promoter and other appropriate control signals, and introducing the expression cassette into a cell. More than one of the enzymes can be expressed in the same host cells using a variety of methods. For example, a single extrachromosomal vector can include multiple expression cassettes or more that one compatible extrachromosomal vector can be used maintain an expression cassette in a host cell. Expression cassettes can also be inserted into a host cell chromosome, using methods known to those of skill in the art. Those of skill will recognize that combinations of expression cassettes in extrachromosomal vectors and expression cassettes inserted into a host cell chromosome can also be used. Other modification of the host cell, described in detail below, can be performed to enhance production of the desired oligosaccharide. For example, the microorganism may be LacY+ allowing active transport of lactose. Host cells don't need to be NanT+ since activated sialylic acid is produced internally with the method according to the invention.

The recombinant cells of the invention are generally microorganisms, such as, for example, yeast cells, bacterial cells, or fungal cells. Examples of suitable cells include, for example, *Azotobacter sp.* (*e.g., A. vinelandii*)*, Pseudomonas sp., Rhizobium sp., Erwinia sp., Bacillus sp., Streptomyces sp., Escherichia sp.* (*e.g., E. coli*), and *Klebsiella sp.,* among many others. The cells can be of any of several genera, including Saccharomyces (*e.g., S. cerevisiae*), Candida (*e.g., C. utilis, C. parapsilosis, C. krusei, C*. *versatilis, C. lipolytica, C. zeylanoides, C. guilliermondii, C. albicans, and C. humicola*), Pichia (*e.g., P.ƒarinosa and P. ohmeri*), Torulopsis (*e.g., T. candida, T. sphaerica, T. xylinus, T.famata, and T. versatilis*), Debaryomyces (*e.g., D. subglobosus, D. cantarellii, D. globosus, D. hansenii, and D. japonicus*), Zygosaccharomyces (*e.g., Z. rouxii and Z. bailii*), Kluyveromyces (*e.g., K. marxianus*), Hansenula (*e.g., H. anomala and H. jadinii*), and Brettanomyces (*e.g., B. lambicus and B. anomalus*).

Promoters for use in *E. coli* include the T7, trp, or lambda promoters. A ribosome binding site and preferably a transcription termination signal are also provided. For expression of heterologous proteins in prokaryotic cells other than *E. coli,* a promoter that functions in the particular prokaryotic species is required. Such promoters can be obtained from genes that have been cloned from the species, or heterologous promoters can be used. For example, the hybrid trp-lac promoter functions in *Bacillus* in addition to *E. coli.* Methods of transforming prokaryotes other than E. coli are well known. For example, methods of transforming Bacillus species and promoters that can be used to express proteins are taught in U.S. Patent No. 6,255,076 and U.S. Patent No. 6,770,475.

In yeast, convenient promoters include GAL1-10 (Johnson and Davies (1984) Mol. Cell. Biol. 4:1440-1448) ADH2 (Russell et al. (1983) J. Biol. Chem. 258:2674-2682), PHO5 (EMBO J. (1982) 6:675-680), and MFα (Herskowitz and Oshima (1982) in The Molecular Biology of the Yeast Saccharomyces (eds. Strathern, Jones, and Broach) Cold Spring Harbor Lab., Cold Spring Harbor, N.Y., pp. 181-209). Another suitable promoter for use in yeast is the ADH2/GAPDH hybrid promoter as described in Cousens et al., Gene 61:265-275 (1987). For filamentous fungi such as, for example, strains of the fungi Aspergillus (McKnight et al., U.S. Patent No. 4,935,349), examples of useful promoters include those derived from *AsPergillus nidulans* glycolytic genes, such as the ADH3 promoter (McKnight et al., EMBO J. 4: 2093 2099 (1985)) and the tpiA promoter. An example of a suitable terminator is the ADH3 terminator (McKnight *et al*.).

In some embodiments, the polynucleotides are placed under the control of an inducible promoter, which is a promoter that directs expression of a gene where the level of expression is alterable by environmental or developmental factors such as, for example, temperature, pH, anaerobic or aerobic conditions, light, transcription factors and chemicals. Such promoters are referred to herein as "inducible" promoters, which allow one to control the timing of expression of the glycosyltransferase or enzyme involved in nucleotide sugar synthesis. For *E. coli* and other bacterial host cells, inducible promoters are known to those of skill in the art. These include, for example, the *lac* promoter. A particularly preferred inducible promoter for expression in prokaryotes is a dual promoter that includes a *tac* promoter component linked to a promoter component obtained from a gene or genes that encode enzymes involved in galactose metabolism (*e*.*g*., a promoter from a UDPgalactose 4-epimerase gene (*gal*E)).

Inducible promoters for other organisms are also well known to those of skill in the art. These include, for example, the arabinose promoter, the lacZ promoter, the metallothionein promoter, and the heat shock promoter, as well as many others.

The construction of polynucleotide constructs generally requires the use of vectors able to replicate in bacteria. A plethora of kits are commercially available for the purification of plasmids from bacteria. For their proper use, follow the manufacturer's instructions *(see,* for example, EasyPrepJ, FlexiPrepJ, both from Pharmacia Biotech; StrataCleanJ, from Stratagene; and, QIAexpress Expression System, Qiagen). The isolated and purified plasmids can then be further manipulated to produce other plasmids, and used to transfect cells. Cloning in *Streptomyces* or *Bacillus* is also possible.

Selectable markers are often incorporated into the expression vectors used to construct the cells of the invention. These genes can encode a gene product, such as a protein, necessary for the survival or growth of transformed host cells grown in a selective culture medium. Host cells not transformed with the vector containing the selection gene will not survive in the culture medium. Typical selection genes encode proteins that confer resistance to antibiotics or other toxins, such as ampicillin, neomycin, kanamycin, chloramphenicol, or tetracycline. Alternatively, selectable markers may encode proteins that complement auxotrophic deficiencies or supply critical nutrients not available from complex media, *e.g*., the gene encoding D-alanine racemase for Bacilli. Often, the vector will have one selectable marker that is functional in, *e*.*g*., *E. coli,* or other cells in which the vector is replicated prior to being introduced into the target cell. A number of selectable markers are known to those of skill in the art and are described for instance in Sambrook *et al., supra.* A preferred selectable marker for use in bacterial cells is a kanamycin resistance marker (Vieira and Messing, Gene 19: 259 (1982)). Use of kanamycin selection is advantageous over, for example, ampicillin selection because ampicillin is quickly degraded by β-lactamase in culture medium, thus removing selective pressure and allowing the culture to become overgrown with cells that do not contain the vector.

Construction of suitable vectors containing one or more of the above listed components employs standard ligation techniques as described in the references cited above. Isolated plasmids or DNA fragments are cleaved, tailored, and re-ligated in the form desired to generate the plasmids required. To confirm correct sequences in plasmids constructed, the plasmids can be analyzed by standard techniques such as by restriction endonuclease digestion, and/or sequencing according to known methods. Molecular cloning techniques to achieve these ends are known in the art. A wide variety of cloning and *in vitro* amplification methods suitable for the construction of recombinant nucleic acids are well-known to persons of skill.

A variety of common vectors suitable for constructing the recombinant cells of the invention are well known in the art. For cloning in bacteria, common vectors include pBR322 derived vectors such as pBLUESCRIPT™, and λ-phage derived vectors. In yeast, vectors include Yeast Integrating plasmids (*e*.*g*., YIp5) and Yeast Replicating plasmids (the YRp series plasmids) and pGPD-2.

The methods for introducing the expression vectors into a chosen host cell are not particularly critical, and such methods are known to those of skill in the art. For example, the expression vectors can be introduced into prokaryotic cells, including *E*. *coli,* by calcium chloride transformation, and into eukaryotic cells by calcium phosphate treatment or electroporation. Other transformation methods are also suitable.

### EXAMPLES

### Example 1: construction of the nanKETA mutant

The *nanA, nanK, nanT* mutant strain ZLKA was constructed from *Escherichia coli* K12 strain DC (Dumon *et al.,* 2005). In *E. coli* K12 the *nanA nanK* and *nanT* genes are clustered in the same region of the *E. coli* chromosome together with the *nanE* gene which encodes the ManNac kinase activity. These four genes were simultaneously deleted by removing a 3.339 kb segment in the chromosomal DNA using the previously described one-step procedure that employs PCR primers to provide the homology to the targeted sequence (Datsenko & Wanner, 2000). The sequence of the upstream primer was
5GCAATTATTGATTCGGCGGATGGTTTGCCGATGGTGGTGTAGGCTGGAGCTGCTT C (SEQ ID NO 1)
and the sequence of the downstream primer was
5'CTCGTCACCCTGCCCGGCGCGCGTGAAAATAGTTTTCGCATATGAATATCCTCCTT AG (SEQ ID NO 2)

### Example 2: Cloning of neuBCA genes

A 2.995 DNA fragment containing the sequence of the genes *neuBCA* was amplified by PCR using the genomic DNA of *Campylobacter jejuni* strain ATCC 43438 as a template. A *KpnI* site was added to the left primer
(5'GGTACCTAAGGAGGAAAATAAATGAAAGAAATAAAAATACAA) (SEQ ID NO 3)
and a *XhoI* site (5'CTCGAGTTAAGTCTCTAATCGATTGTTTTCCAATG) (SEQ ID NO 4)
was added to the right primer The amplified fragment was first cloned into pCR4Blunt-TOPO vector (Invitrogen) and then sub-cloned into the *KpnI* and *XhoI* sites of pBBR1-MCS3 vector to form pBBR3-SS.

### Example 3: Construction of plasmids expressing the neuBC genes and an inactive neuA gene

In plasmid pBBR3-SS the *neuA* gene is located downstream the *neuC* gene. A 0.4 k DNA fragment located in the *neuA* gene sequence was excised from pBBR3-SS par digestion with *BsaBI* and *SmaI.* After ligation the resulting plasmid contained a truncated inactive *neuA* gene which was called pBBR3-neuBC.

To increase their expression level, the *neuBC* gene were subcloned from the low copy number plasmid pBBR3-neuBC into the *KpnI* and *XbaI* sites of high copy number plasmid pBluescript II KS, yielding pBS-neuBC.

### Example 4: Production of Neu5Ac by high cell density culture of nanKETA mutant expressing neuBC genes

The strains DC0, SI1 and SI2 were constructed by transforming the *nanKETA* mutant host strain ZLKA described in example 1 with plasmids pBBR3-SS, pBBR3-neuBC and pBS-neuBC respectively. Cultures were carried out in 3-litre reactors containing 1.5 litre of mineral culture medium.The temperature was maintained at 34°C and the pH was regulated to 6.8 with 14 % NH₄OH. As previously described *(Priem et al.,* 2002), the high cell density culture consisted of three phases: an exponential growth phase which started with the inoculation of the fermenter and lasted until exhaustion of the carbon substrate (glucose 17.5g.L⁻¹), a 5-h fed-batch with a high glycerol feeding rate of 5.5 g.L⁻¹h⁻¹ and a 19-h fed-batch phase with a lower glycerol feeding rate of 3.15 g.L⁻¹h⁻¹. The inducer (IPTG 50 mg) was added at the end of the exponential phase. Colorimetric quantification of Neu5Ac showed that strain DC0 which expressed the three gene *neuBCA* did not produce Neu5Ac (Table 2). By contrast the two strains SI1 and SI2 which expressed a truncated form of the neuA gene produced large amounts of Neu5Ac which was mainly recovered in the extracellular fraction. Neu5Ac production was three times higher in strain SI2 than in strain SI1 in reason of a higher expression of the *neuBC* genes in the high copy number plasmid pBS-neuBC.

**Table 2. Colorimetric quantification of sialic acid in intracellular and extracellular fractions of high cell density cultures of strains genetically engineered for the production of Neu5Ac**

| Strain | plasmid | hetrologous genes | Neu5Ac concentration (g.l⁻¹) | |
|---|---|---|---|---|
| | | expressed | intracellular | extracellular |
| DC0 | pBBR3-SS | *neuBCA* | 0 | 0 |
| SI1 | pBBR3-neuBC | *neuBC* | 0.57 | 3.35 |
| SI2 | pBS-neuBC | *neuBC* | 1.1 | 11.3 |

| | | | | |
|---|---|---|---|---|
| Total sialic acid was quantified by the diphenylamine method (Werner & Odin, 1952) after 40 hours of culture. | | | | |

### Example 5: optimisation of Neu5Ac production.

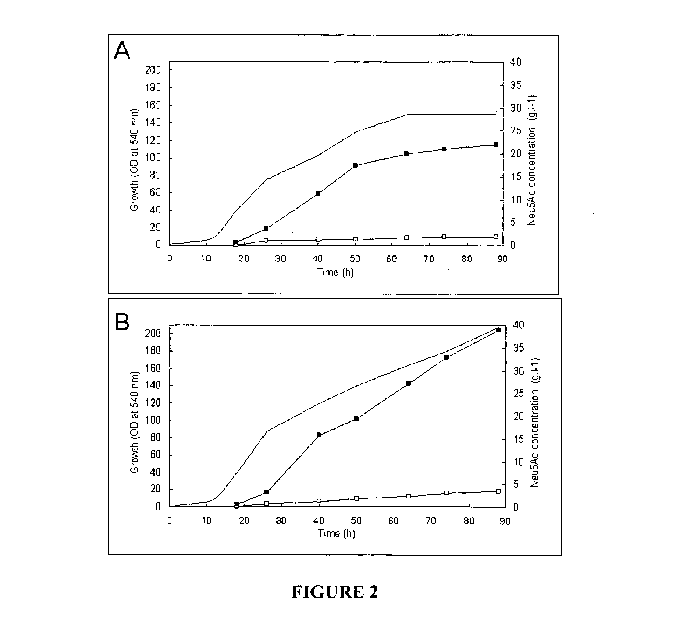

Further optimization of Neu5Ac production was carried out with strain SI2 carrying the pBS-neuBC plasmid. In order to increase the Neu5Ac yield, the fermentation time course was first extended up to 84 hours while maintaining a constant glycerol feeding rate of 3.15 g.L⁻¹h⁻¹. This resulted in final extracellular concentration of 22 g.L⁻¹ (Figure 2A). However the Neu5Ac production rate significantly decreased after 50 hours of culture. Increasing the glycerol feeding rate up to 4.2 g.L⁻¹h⁻¹ enable the Neu5Ac to be produced for a longer period and a final extracellular concentration of 39 g.L⁻¹ was obtained.

### Example 6: Purification of Neu5Ac

At the end of the culture of strain SI2 (Figure 2A), the cell were eliminated by centrifugation and Neu5Ac was purified from the supernatant by direct crystallization as previously described (Maru *et al.,* 1998). The supernatant (2 liter) was concentrated to volume of 140 ml and crystallization was initiated by adding 700 ml of glacial acetic acid. The mixture was incubated overnight at 4 °C and Neu5Ac crystals were recovered by filtration. After were washing with 1 liter of cold isopropanol and drying under vacuum, 33.4 g of crystallized Neu5Ac were obtained. Identification of Neu5Ac was confimed by mass spectrum analysis of solubilised crystals in the negative mode (ESI⁻) which showed a single peak at m/z 308 corresponding to the quasimolecular ions [M-H]⁻ derived from Neu5Ac.

### Example 7: Effect of nanK and nanT knockout on sialic acid production

Strain JM 107 containing the intact sialic acid operon was obtained from the German collection of microorganism (DSM 3950). Construction of TA01,which is a *nanA⁻* derivative of strain JM107, was previously described (Antoine *et al.,* 2003). The *nanKETA* strain S17 was constructed by disrupting the entire sialic acid gene cluster in strain JM 107 as described in example 1. The strain SI6 was constructed by deleting the nanK gene in the TA01 strain by removing a 1.11 kb segment in the chromosomal DNA using the previously described one-step procedure that employs PCR primers to provide the homology to the targeted sequence (Datsenko & Wanner, 2000). The sequence of the upstream primer was
5'GGCAGAACAGGCGGGCGCGGTTGCCATTCGCATTGAAGGTGTAGGCTGGAGCTG CTTC (SEQ ID NO 1) and the sequence of the downstream primer was
5'CTCGTCACCCTGCCCGGCGCGCGTGAAAATAGTTTTCGCATATGAATATCCTCCTT AG (SEQ ID NO 2)

Strain JM107, TA01, SI6 and SI7 were transformed with the pBS-neuBC plasmid described in example 3. Sialic acid production was then investigated with the resulting transformants in shake flask cultures using a mineral culture medium supplemented with glycerol (5 g.l⁻¹) as the carbon and energy source. As shown in table 3, the *nanA* mutant strain TA01 produced very little amount of sialic acid. By contrast the strain SI6 and SI7 which both had the additional *nanK* mutation produced important amounts of sialic acid. However the strain SI6 which had a functional sialic acid permease (NanT) produced two times less sialic acid than the *nanKTA* mutant strain SI7

These results indicate that the *nanK* disruption is necessary to obtain an efficient production of sialic acid. On the contrary, albeit the *nanT* disruption improves the productivity and allows sialic acid to be mainly recovered in the extracellular medium, a significant production of sialic acid can still be achieved with a strain expressing a functional sialic acid permease.

**Table 3. Sialic acid production by strains harboring different deletions in the sialic acid gene cluster and expressing the neuBC genes**

| Host strain | deleted genes | Neu5Ac production (mg/l) | | |
|---|---|---|---|---|
| | | intracellular | extracellular | total |
| JM107 | none | nd | nd | nd |
| TA01 | *nanA* | 2.5 | nd | 2.5 |
| SI6 | *nanK nanA* | 43 | 48 | 91 |
| SI7 | *nanK nanE nanT nanA* | 29 | 178 | 207 |

| | | | | |
|---|---|---|---|---|
| nd : not detectable Sialic acid production was determined colorimetrically by the diphenylamine method after 24 hours of culture | | | | |

### REFERENCES

Annunziato, P. W., Wright, L. F., Vann, W. F. & Silver, R. P. (1995). Nucleotide sequence and genetic analysis of the neuD and neuB genes in region 2 of the polysialic acid gene cluster of Escherichia coli K1.J Bacteriol 177, 312-319.
Antoine, T., Priem, B., Heyraud, A., Greffe, L., Gilbert, M., Wakarchuk, W. W., Lam, J. S. & Samain, E. (2003). Large-scale in vivo synthesis of the carbohydrate moieties of gangliosides GM 1 and GM2 by metabolically engineered Escherichia coli. Chembiochem 4, 406-412.
Blayer, S., Woodley, J. M., Dawson, M. J. & Lilly, M. D. (1999). Alkaline biocatalysis for the direct synthesis of N-acetyl-D-neuraminic acid (Neu5Ac) from N-acetyl-D-glucosamine (GlcNAc). Biotechnol Bioeng 66, 131-136.
Datsenko, K. A. & Wanner, B. L. (2000). One-step inactivation of chromosomal genes in Escherichia coli K-12 using PCR products. Proc Natl Acad Sci U S A 97, 6640-6645.
Dumon, C., Bosso, C., Utille, J. P., Heyraud, A. & Samain, E. (2005). Production of Lewis x Tetrasaccharides by Metabolically Engineered Escherichia coli. Chembiochem 7,359-365.
Kalivoda, K. A., Steenbergen, S. M., Vimr, E. R. & Plumbridge, J. (2003). Regulation of sialic acid catabolism by the DNA binding protein NanR in Escherichia coli. J Bacteriol 185, 4806-4815.
Koketsu, M., Juneja, L. R., Kawanami, H., Kim, M. & Yamamoto, T. (1992). Preparation of N-acetylneuraminic acid from delipidated egg yolk. Glycoconj J 9, 70-74.
Kornfeld, S., Kornfeld, R., Neufeld, E. F. & O'Brien, P. J. (1964). The Feedback Control of Sugar Nucleotide Biosynthesis in Liver. Proc Natl Acad Sci U S A 52, 371-379.
Kragl, U., Gygas, D., Ghisalba, O. & Wandrey, C. (1991). Enzymatic two-step synthesis of N-acetylneuraminic acid in the enzyme membrane reactor. AngewChem Int Ed Engl 30, 827-828.
Lee, J., Yi, J., Lee, S., Takahashi, S. & Kim, B. (2004). Production of N-acetylneuraminic acid from N acetylglucosamineand pyruvate using recombinant human renin binding protein and sialic aldolase in one pot. Enzyme Microb Technol 35, 121-125.
Mahmoudian, M., Noble, D., Drake, C. S., Middleton, R. F., Montgomery, D. S., Piercey, J. E., Ramlakhan, D., Todd, M. & Dawson, M. J. (1997). An efficient process for production of N-acetylneuraminic acid using N-acetylneuraminic acid aldolase. Enzyme Microb Technol 20, 393-400.
Martin, J. E., Tanenbaum, S. W. & Flashner, M. (1977). A facile procedure for the isolation of N-acetylneuramic acid from edible bird's-nest. Carbohydr Res 56, 423-425.
Martinez, J., Steenbergen, S. & Vimr, E. (1995). Derived structure of the putative sialic acid transporter from Escherichia coli predicts a novel sugar permease domain. J Bacteriol 177, 6005-6010.
Maru, I., Ohta, Y., Murata, K. & Tsukada, Y. (1996). Molecular cloning and identification of N-acyl-D-glucosamine 2-epimerase from porcine kidney as a renin-binding protein. J Biol Chem 271, 16294-16299.
Maru, I., Ohnishi, J., Ohta, Y. & Tsukada, Y. (1998). Simple and large-scale production of N-acetylneuraminic acid from N-acetyl-D-glucosamine and pyruvate using N-acyl-D-glucosamine 2-epimerase and N-acetylneuraminate lyase. Carbohydr Res 306, 575-578.
Plumbridge, J. & Vimr, E. (1999). Convergent pathways for utilization of the amino sugars N-acetylglucosamine, N-acetylmannosamine, and N-acetylneuraminic acid by Escherichia coli. J Bacterio/ 181, 47-54.
Priem, B., Gilbert, M., Wakarchuk, W. W., Heyraud, A. & Samain, E. (2002). A new fermentation process allows large-scale production of human milk oligosaccharides by metabolically engineered bacteria. Glycobiology 12, 235-240.
Uchida, Y., Tsukada, Y. & Sugimori, T. (1973). Improved microbiol production of colominic acid, a homopolymer of N-acetylneuraminic acid. Agr Biol Chem 37, 2105-2110.
Vann, W. F., Tavarez, J. J., Crowley, J., Vimr, E. & Silver, R. P. (1997). Purification and characterization of the Escherichia coli K1 neuB gene product N-acetylneuraminic acid synthetase. Glycobiology 7, 697-701.
Vann, W. F., Daines, D. A., Murkin, A. S., Tanner, M. E., Chaffin, D.O., Rubens, C. E., Vionnet, J. & Silver, R. P. (2004). The NeuC protein of Escherichia coli K1 is a UDP N-acetylglucosamine 2-epimerase. J Bacteriol 186, 706-712.
Varki, A. (1993). Biological roles of oligosaccharides: all of the theories are correct. Glocobiology 3, 97-130.
Vimr, E. R. & Troy, F. A. (1985). Identification of an inducible catabolic system for sialic acids (nan) in Escherichia coli. J Bacteriol 164, 845-853.
Werner, I. & Odin, L. (1952). On the presence of sialic acid in certain glycoproteins and in gangliosides. Acta Soc Med Ups 57, 230-241.

## Claims

1. A method for producing sialic acid, comprising the step of culturing a microorganism in a culture medium, wherein said microorganism comprises heterologous genes encoding a sialic acid synthase (NeuB), a UDP-GlcNAc epimerase (NeuC), said micro-organism being devoid of a gene encoding CMP-Neu5Ac synthase (NeuA) or wherein a gene encoding CMP-Neu5Ac synthase (NeuA) has been inactivated or deleted; and wherein endogenous genes coding for sialic acid aldolase (NanA), for sialic acid transporter (NanT), and optionally for ManNac kinase (NanK), have been deleted or inactivated.

2. The method according to claim 1, wherein degradation of Neu5Ac and ManNAc is prevented by disrupting the *nanA* and *nanK* genes.

3. The method according to claim 2, further comprising deletion or inactivation of the *nanT* gene.

4. The method according to claim 1, wherein it comprises removing the operon including *nanT, nanA, nanK* and *nanE* genes *(nanKEAT-).*

5. The method according to claim 1, wherein it comprises removing the operon including *nanT, nanA, nanE* genes *(nanEAT-),* except the *nanK* gene.

6. The method according to claim 1, wherein the heterologous genes originate from *E. coli, Neisseria,* or *Campylobacter* species.

7. The method according to claim 5 or 6, wherein the NeuB and NeuC genes are isolated from *C. jejuni* strain ATCC Accession No. 43438.

8. The method according to claim 1, wherein said microorganism is cultured in conditions comprising an exponential growth phase which starts with the inoculation of the fermenter and last until exhaustion of the carbon substrate.

9. The method according to claim 8, wherein the carbon substrate is glucose.

10. The method according to claim 8, wherein said microorganism is cultured after said exponential phase in a 40 hours to at least 75,100 or 150 hours fed-batch with a high glycerol feeding rate of between 4 g.L⁻¹h⁻¹ to 6 g.L⁻¹h⁻¹.

11. The method according to claim 1 further comprising steps of purification such as removal of cells by centrifugation, followed by crystallization and filtration.

12. A microorganism comprising heterologous genes encoding a sialic acid synthase (NeuB), a UDP-GlcNAc epimerase (NeuC), said micro-organism being devoid of a gene encoding CMP-Neu5Ac synthase (NeuA) or wherein a gene encoding CMP-Neu5Ac synthase (NeuA) has been inactivated or deleted; and wherein endogenous genes coding for sialic acid aldolase (NanA), for sialic acid transporter (NanT), and optionally for ManNac kinase (NanK), have been deleted or inactivated.

13. A cell culture medium comprising a microorganism according to claim 12 and sialic acid produced therefrom which concentration ranges from 10 to 50 g/l in said culture medium.

## Patentansprüche

1. Verfahren zum Herstellen von Sialinsäure, welches den Schritt umfasst, einen Mikroorganismus in einem Kulturmedium zu kultivieren, wobei der Mikroorganismus heterologe Gene, welche eine Sialinsäuresynthase (NeuB), eine UDP-GlcNAc-Epimerase (NeuC) kodieren, umfasst, wobei dem Mikroorganismus ein Gen, welches CMP-Neu5Ac-Synthase (NeuA) kodiert, fehlt oder in welchem ein Gen, welches CMP-Neu5Ac-Synthase (NeuA) kodiert, inaktiviert oder deletiert worden ist; und in welchem endogene Gene, welche Sialinsäurealdolase (NanA), Sialinsäure-Transporter (NanT) und gegebenenfalls ManNac-Kinase (NanK) kodieren, deletiert oder inaktiviert worden sind.

2. Verfahren nach Anspruch 1, wobei ein Abbau von Neu5Ac und ManNAc verhindert wird, indem die *nanA-* und *nanK*-Gene zerstört werden.

3. Verfahren nach Anspruch 2, welches ferner eine Deletion oder Inaktivierung des *nanT-*Gens umfasst.

4. Verfahren nach Anspruch 1, wobei es das Entfernen des *nanT , nanA-, nanK-*und *nanE*-Gene umfassenden Operons *(nanKEAT-)* umfasst.

5. Verfahren nach Anspruch 1, wobei es das Entfernen des *nanT-, nanA-, nanE-*Gene umfassenden Operons mit Ausnahme des nanK-Gens (*nanEAT*-) umfasst.

6. Verfahren nach Anspruch 1, wobei die heterologen Gene aus *E*. *coli-, Neisseria-* oder *Campylobacter*-Spezies stammen.

7. Verfahren nach Anspruch 5 oder 6, wobei die NeuB- und NeuC-Gene aus dem *C*. *jejuni*-Stamm ATCC Accession No. 43438 isoliert werden.

8. Verfahren nach Anspruch 1, wobei der Mikroorganismus unter Bedingungen, welche eine exponentielle Vermehrungsphase, die mit dem Animpfen des Fermenter beginnt und bis zur Erschöpfung des Kohlenstoffsubstrats dauert, umfassen, kultiviert wird.

9. Verfahren nach Anspruch 8, wobei das Kohlenstoffsubstrat Glucose ist.

10. Verfahren nach Anspruch 8, wobei der Mikroorganismus nach der exponentiellen Phase in einem 40-stündigen bis mindestens 75-, 100- oder 150-stündigen Fed-Batch-Verfahrensabschnitt mit einer hohen Glycerol-Zufütterungsrate zwischen 4 g·l⁻¹h⁻¹ bis 6 g·l⁻¹h⁻¹ kultiviert wird.

11. Verfahren nach Anspruch 1, welches ferner Reinigungsschritte, wie eine Entfernung von Zellen durch Zentrifugation, gefolgt von Kristallisierung und Filtration, umfasst.

12. Mikroorganismus, umfassend heterologe Gene, welche eine Sialinsäuresynthase (NeuB), eine UDP-GlcNAc-Epimerase (NeuC) kodieren, wobei dem Mikroorganismus ein Gen, welches CMP-Neu5Ac-Synthase (NeuA) kodiert, fehlt oder in welchem ein Gen, welches CMP-Neu5Ac-Synthase (NeuA) kodiert, inaktiviert oder deletiert worden ist; und in welchem endogene Gene, welche Sialinsäurealdolase (NanA), Sialinsäure-Transporter (NanT) und gegebenenfalls ManNac-Kinase (NanK) kodieren, deletiert oder inaktiviert worden sind.

13. Zellkulturmedium, welches einen Mikroorganismus nach Anspruch 12 und ausgehend von diesem hergestellte Sialinsäure, deren Konzentration von 10 bis 50 g/l in dem Kulturmedium reicht, umfasst.

## Revendications

1. Procédé de production d'acide sialique, comprenant l'étape consistant à cultiver un micro-organisme dans un milieu de culture, où ledit micro-organisme comprend des gènes hétérologues codant pour une acide sialique synthase (NeuB), une UDP-GlcNAc épimérase (NeuC), ledit micro-organisme étant dépourvu de gène codant pour une CMP-Neu5Ac synthase (NeuA) ou bien dans lequel un gène codant pour une CMP-Neu5Ac synthase (NeuA) a été inactivé ou délété ; et dans lequel les gènes endogènes codant pour une acide sialique aldolase (NanA), pour un transporteur d'acide sialique (NanT), et éventuellement pour une ManNac kinase (NanK), ont été délétés ou inactivés.

2. Procédé selon la revendication 1, dans lequel la dégradation de la Neu5Ac et de la ManNAc est prévenue par la rupture des gènes *nanA* et *nanK*.

3. Procédé selon la revendication 2, comprenant en outre la délétion ou l'inactivation du gène *nanT*.

4. Procédé selon la revendication 1, dans lequel il est compris l'élimination de l'opéron comprenant les gènes *nanT, nanA, nanK* et *nanE* (*nanKEAT*-).

5. Procédé selon la revendication 1, dans lequel il est compris l'élimination de l'opéron comprenant les gènes *nanT, nanA, nanE* (*nanEAT*-), à l'exception du gène *nanK*.

6. Procédé selon la revendication 1, dans lequel les gènes hétérologues proviennent des espèces *E. coli, Neisseria* ou *Campylobacter.*

7. Procédé selon la revendication 5 ou 6, dans lequel les gènes NeuB et NeuC sont isolés de la souche ATCC de *C. jejuni* avec le No. d'accession 43438.

8. Procédé selon la revendication 1, dans lequel ledit micro-organisme est cultivé dans des conditions comprenant une phase de croissance exponentielle qui démarre avec l'inoculation du fermenteur et dure jusqu'à l'épuisement du substrat carboné.

9. Procédé selon la revendication 8, dans lequel le substrat carboné est du glucose.

10. Procédé selon la revendication 8, dans lequel ledit micro-organisme est cultivé après ladite phase exponentielle dans un mode alimenté de 40 heures à au moins 75, 100 ou 150 heures avec une vitesse d'alimentation riche en glycérol située entre 4 g.l⁻¹h⁻¹ et 6 g.l⁻¹h⁻¹.

11. Procédé selon la revendication 1, comprenant en outre l'étape de purification, telle que l'élimination des cellules par centrifugation, suivie des étapes de cristallisation et de filtration.

12. Micro-organisme comprenant des gènes hétérologues codant pour une acide sialique synthase (NeuB), une UDP-GlcNAc épimérase (NeuC), ledit micro-organisme étant dépourvu de gène codant pour une CMP-Neu5Ac synthase (NeuA) ou bien dans lequel un gène codant pour une CMP-Neu5Ac synthase (NeuA) a été inactivé ou délété ; et dans lequel les gènes endogènes codant pour une acide sialique aldolase (NanA), pour un transporteur d'acide sialique (NanT), et éventuellement pour une ManNac kinase (NanK), ont été délétés ou inactivés.

13. Milieu de culture cellulaire comprenant un micro-organisme selon la revendication 12 et de l'acide sialique produit à partir de ce dernier dont la concentration est située dans la plage de 10 à 50 g/l dans ledit milieu de culture.
